(19)

Europäisches
Patentamt
European
Patent Office
Office européen
des brevets

(11) **EP 3 553 518 A1**

(12) **EUROPEAN PATENT APPLICATION**

(43) Date of publication:
   **16.10.2019 Bulletin 2019/42**

(51) Int Cl.:
   ***G01N 33/573*** (2006.01)   ***G01N 33/68*** (2006.01)
   ***G06F 19/10*** (2011.01)   ***G16H 50/30*** (2018.01)

(21) Application number: **18166975.5**

(22) Date of filing: **12.04.2018**

(84) Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**
   Designated Extension States:
   **BA ME**
   Designated Validation States:
   **KH MA MD TN**

(71) Applicant: **Koninklijke Philips N.V.
   5656 AE Eindhoven (NL)**

(72) Inventors:
   • **KOOIJMAN, Gerben
     5656 AE Eindhoven (NL)**
   • **RMAILE, Amir Hussein
     5656 AE Eindhoven (NL)**
   • **GLASSE, Carl
     5656 AE Eindhoven (NL)**

   • **DE JAGER, Marinus Karel Johannes
     5656 AE Eindhoven (NL)**
   • **CHAPPLE, Iain Leslie Campbell
     5656 AE Eindhoven (NL)**
   • **GRANT, Melissa Mackay
     5656 AE Eindhoven (NL)**
   • **PRESHAW, Philip
     5656 AE Eindhoven (NL)**
   • **TAYLOR, John
     5656 AE Eindhoven (NL)**
   • **VAN HARTSKAMP, Michael Alex
     5656 AE Eindhoven (NL)**

(74) Representative: **de Haan, Poul Erik et al
   Philips International B.V.
   Philips Intellectual Property & Standards
   High Tech Campus 5
   5656 AE Eindhoven (NL)**

(54) **PERIODONTITIS DIAGNOSTIC METHODS, USES AND KITS**

(57)    Disclosed is an *in vitro* method for assessing whether a human patient has periodontitis. The method is based on the insight to determine biomarker proteins. Accordingly, in a sample of saliva a patient suffering from periodontitis, the concentrations are measured of two or more of the proteins Matrix metalloproteinase-8 (MMP-8), Matrix metalloproteinase-9 (MMP-9), Pyruvate Kinase (PK) and S100 calcium-binding protein A8 (S100A8). Based on the concentrations as measured, a value is determined reflecting the joint concentrations for said proteins. This value is compared with a threshold value reflecting in the same manner the joint concentrations associated with periodontitis. The comparison allows assessing whether the testing value is indicative of the presence of periodontitis in said patient. Thereby, typically, a testing value reflecting a joint concentration below the joint concentration reflected by the threshold value is indicative for absence of periodontitis in said patient, and a testing value reflecting a joint concentration at or above the joint concentration reflected by the threshold value, is indicative for periodontitis in said patient.

FIG. 1

EP 3 553 518 A1

**Description**

FIELD OF THE INVENTION

The invention is in the field of oral care, and pertains to saliva-based diagnostics of periodontal disease. Particularly, the invention pertains to a kit, use and method for diagnosing periodontitis.

BACKGROUND OF THE INVENTION

Gum inflammation, or gingivitis, is a non-destructive periodontal disease caused mainly by the adherence of dental bacterial biofilms, or dental plaque, to the tooth surface. If not detected and treated, the reversible gingivitis usually leads to the inflammation of the tissues surrounding the tooth (i.e. periodontal tissues), a condition defined as periodontitis, which is irreversible and causes tissue destruction and alveolar bone loss, and ultimately results in the loss of teeth. During the progression of gum disease, there are usually clinical signs and symptoms associated with it, such as the swelling of the gums, the change in color from pink to dark red, the bleeding of the gums, bad breath, and the gums becoming more tender or painful to touch.

Periodontitis is a chronic multifactorial inflammatory disease caused by oral microorganisms and characterized by progressive destruction of the hard (bone) and soft (periodontal ligament) tissues, ultimately leading to tooth mobility and loss. This is to be distinguished from gingivitis which is a reversible infection and inflammation of the gum tissues. Inflammatory periodontitis is one of the most prevalent chronic human diseases and a major cause of adult tooth loss. In addition to the substantial negative impact of periodontitis on oral health, there is also mounting evidence that periodontitis has systemic consequences and that it is a risk factor for several systemic diseases, including heart diseases (e.g. atherosclerosis, stroke), diabetes, pregnancy complications, rheumatoid arthritis and respiratory infections.

Early and accurate diagnosis of periodontal disease, thus, is important from both an oral and overall health perspective.

Periodontal diseases are still poorly diagnosed in general dental practice, resulting in relatively low rates of therapeutic intervention and significant amounts of untreated cases. Current diagnosis relies on imprecise, subjective clinical examination of oral tissue condition (color, swelling, extent of bleeding on probing, probing pocket depth; and bone loss from oral x-rays) by dental professionals. These conventional methods are time consuming, and some of the techniques used (pocket-depth, x-ray) reflect historic events, such as past disease activity, rather than current disease activity or susceptibility to further disease. Hence, more objective, faster, accurate, easier-to-use diagnostics which preferably may also be performed by non-specialists are desirable. Thereby it is desirable to measure current disease activity, and possibly a subject's susceptibility to further periodontal disease.

Saliva or oral fluids have long been advocated as a diagnostic fluid for oral and general diseases, and with the advent of miniaturized biosensors, also referred to as lab-on-a-chip, point of care diagnostics for rapid chair-side testing have gained greater scientific and clinical interest. Especially for periodontal disease detection, inflammatory biomarkers associated with tissue inflammation and breakdown may easily end up in saliva due to proximity, suggesting saliva has strong potential for periodontal disease detection. Indeed, this area thus has gained significant interest and encouraging results have been presented. For example, Ramseier et al (J Periodontol. 2009 Mar;80(3):436-46) identified host- and bacterially derived biomarkers correlated with periodontal disease. However, no definite test has emerged yet.

Biomarkers represent biological indicators that underpin clinical manifestations, and as such are objective measures by which to diagnose clinical outcomes of periodontal disease. Ultimately, proven biomarkers could be utilized to assess risk for future disease, to identify disease at the very earliest stages, to identify response to initial therapy, and to allow implementation of preventive strategies.

Previous limitations to the development of point-of-care tests for salivary biomarkers included a lack of technologies that were adaptable to chair-side applications and an inability to analyze multiple biomarkers in individual samples. Also the selection of which multiple biomarkers to include in such a test has not been adequately addressed in the literature nor implemented in practical tests.

It would be desired to provide a simpler process, and particularly a process that requires only that a small saliva sample is taken from a patient, and possibly by the patient him- or herself. It is desired that such a sample be entered into an *in vitro* diagnostic device, which will allow, based on measurement, a classification of the saliva sample such that it can return an indication of the likelihood that the patient is to be classified as suffering from periodontitis.

SUMMARY OF THE INVENTION

In order to better address the foregoing desires, the invention, in one aspect, concerns an *in vitro* method for assessing whether a human patient has periodontitis, the method comprising detecting, in a sample of saliva from said human patient, the concentrations of at least two proteins selected from Matrix metalloproteinase-8 (MMP-8), Matrix metalloproteinase-9 (MMP-9), Pyruvate Kinase (PK) and S100 calcium-binding protein A8 (S100A8); determining a testing value reflecting the joint concentrations determined for said proteins; and comparing the testing value with a threshold value reflecting in the same manner the joint concentrations associated with periodontitis, so as to assess whether the testing value is indicative for periodontitis in said patient.

In another aspect, the invention presents the use of at least two proteins selected from Matrix metalloproteinase-8 (MMP-8), Matrix metalloproteinase-9 (MMP-9), Pyruvate Kinase (PK) and S100 calcium-binding protein A8 (S100A8) in a saliva sample of a human patient, as biomarkers for assessing whether the patient has periodontitis.

Optionally, the age of the patient is also used as a biomarker.

In a further aspect, the invention resides in a system for assessing whether a human patient has periodontitis, the system comprising:

- detection means able and adapted to detect in a sample of saliva of the human patient at least two proteins selected from Matrix metalloproteinase-8 (MMP-8), Matrix metalloproteinase-9 (MMP-9), Pyruvate Kinase (PK) and S100 calcium-binding protein A8 (S100A8); and
- a processor able and adapted to determine from the determined concentrations of said proteins an indication of the patient having periodontitis.

The system optionally contains a data connection to an interface, particularly a graphical user interface, capable of presenting information, preferably also capable of putting in information such as the age of the subject, as well as optionally other information such as sex and/or BMI (Body Mass Index), said interface being either a part of the system or a remote interface.

Optionally one or more of the foregoing items, particularly the processor, are enabled to function "in the cloud", i.e., not on a fixed machine, but by means of an internet-based application.

In a still further aspect, the invention provides a kit for detecting at least two biomarkers for periodontitis in a sample of saliva of a human patient, said kit comprising detection reagents for detecting at least two proteins selected from Matrix metalloproteinase-8 (MMP-8), Matrix metalloproteinase-9 (MMP-9), Pyruvate Kinase (PK) and S100 calcium-binding protein A8 (S100A8).

Typically, two or more, or three or more, detection reagents are used, each of which binds a different biomarker. In one embodiment, a first detection reagent is capable of detecting MMP-8 or MMP-9, a second detection reagent is capable of detecting a different protein selected from MMP-8, MMP-9, Pyruvate Kinase (PK) and S100 calcium-binding protein A8 (S100A8), and an optional third detection reagent is capable of detecting a different protein selected from Matrix metalloproteinase-8 (MMP-8), Matrix metalloproteinase-9 (MMP-9), Pyruvate Kinase (PK) and S100 calcium-binding protein A8 (S100A8). In further embodiments, separate detection reagents are provided for each of the protein biomarkers provided in combination in Table 1, below.

In yet another aspect, the invention provides an *in vitro* method for determining a change in status of periodontitis in a human patient over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of at least two proteins selected from Matrix metalloproteinase-8 (MMP-8), Matrix metalloproteinase-9 (MMP-9), Pyruvate Kinase (PK) and S100 calcium-binding protein A8 (S100A8); and comparing the concentrations, whereby a difference in one, two or more of the concentrations, reflects a change in status.

In a further aspect, the invention provides a method of diagnosing whether a human patient has periodontitis, comprising detecting in a sample of saliva of the human patient at least two proteins selected from Matrix metalloproteinase-8 (MMP-8), Matrix metalloproteinase-9 (MMP-9), Pyruvate Kinase (PK) and S100 calcium-binding protein A8 (S100A8); and assessing the presence of periodontitis in the patient on the basis of the concentrations of said proteins in said sample. Optionally, the method of this aspect comprises the further step of treating the periodontitis in the patient.

In yet a further aspect, the invention provides a method of detecting at least two proteins selected from Matrix metalloproteinase-8 (MMP-8), Matrix metalloproteinase-9 (MMP-9), Pyruvate Kinase (PK) and S100 calcium-binding protein A8 (S100A8) in a human patient, comprising:

(a) obtaining a saliva sample from a human patient; and
(b) detecting whether the proteins are present in the sample by contacting the sample with one or more detection reagents for said two or more proteins and detecting binding between each protein and the one or more detection reagents. Typically, there are at least first and second, and sometimes third and fourth, detection reagents as set out elsewhere herein.

BRIEF DESCRIPTION OF THE DRAWINGS

Fig. 1 schematically represents a system for use in the method as described in this disclosure.
Fig.2 is a graph showing the number of studied panels vs. required performance in terms of minimum AUC LOOCV.

DETAILED DESCRIPTION OF THE INVENTION

In a general sense, the invention is based on the judicious insight that periodontitis can be distinguished from a healthy or gingivitis-affected oral cavity with sufficient accuracy based on a measurement of a small number of protein biomarkers. In particular, it has been found that as few as two proteins can serve as a biomarker in a sample of saliva of a human patient, for identifying the presence or absence of periodontitis.

The biomarker proteins are Matrix metalloproteinase-8 (MMP-8), Matrix metalloproteinase-9 (MMP-9), Pyruvate Kinase (PK) and S100 calcium-binding protein A8 (S100A8). Two or more of these proteins are typically used according to the invention. In some embodiments, MMP-8 and/or MMP-9 are present.

The subject's age may optionally be included as an additional marker.

MMPs are a family of enzymes that are responsible for the degradation of extracellular matrix components such as collagen, proteoglycans, laminin, elastin, and fibronectin. They play a central role in the periodontal ligament (PDL) remodelling, both in physiological and pathological conditions. MMP-8, also known as neutrophil collagenase or PMNL collagenase (MNL-CL), is a collagen protease enzyme which is present in the connective tissue of most mammals. MMP-9, also known as 92 kDa type IV collagenase, 92 kDa gelatinase or gelatinase B (GELB), is a matrixin, a class of enzymes that belong to the zinc-metalloproteinases family involved in the degradation of the extracellular matrix.

S100 calcium binding protein A8 (S100A8) is a calcium- and zinc-binding protein which plays a prominent role in the regulation of inflammatory processes and immune response. It can induce neutrophil chemotaxis and adhesion.

Pyruvate kinase catalyses the final step of glycolysis. There are four tissue-specific isozymes of Pyruvate Kinase, each having particular kinetic properties needed for different tissues.

The proteins mentioned above are known in the art. The skilled person is aware of their structure, and of methods to detect them in an aqueous sample, such as a saliva sample.

Table 1 in the Example provides 25 particularly preferred protein biomarker combinations according to the invention. The Table includes age for each combination, but this can optionally be excluded if desired.

A biomarker panel of the invention, in one embodiment, may consist of the protein biomarkers identified. Preferably, a biomarker panel of the invention consists of not more than four of the protein biomarkers identified in the invention, e.g. two, three or four protein biomarkers of the invention. In addition to the biomarker panels of the invention, other biomarkers and or data, such as demographic data (e.g., age, sex) can be included in a set of data applied for the determination of periodontitis. Additional protein biomarkers include IL-1β, HGF, FLC Kappa, FLC Lambda, A1AGP, Hb-beta, Hb-delta, Keratin 4, Profilin, and S100A9. One or more of these additional proteins are included in some of the preferred panels in the Example, below.

IL-1β (Interleukin-1β) is a member of the interleukin 1 family of cytokines. This cytokine is produced by activated macrophages as a proprotein, which is proteolytically processed to its active form by caspase 1 (CASP1/ICE). This cytokine is an important mediator of the inflammatory response, and is involved in a variety of cellular activities, including cell proliferation, differentiation, and apoptosis.

Hepatocyte Growth Factor (HGF) is a paracrine cellular growth, motility and morphogenic factor. It is secreted by mesenchymal cells and targets and acts primarily upon epithelial cells and endothelial cells, but also acts on haemopoietic progenitor cells. HGF has been shown to have a major role in myogenesis and in wound healing. Its ability to stimulate mitogenesis, cell motility, and matrix invasion gives it a central role in angiogenesis, tumorogenesis, and tissue regeneration. HGF stimulates growth of epithelial cells and prevents regeneration of the connective tissue attachment. HGF is known as a serum marker indicating disease activity in various diseases.

Free Light Chain proteins are immunoglobulin light chains. They are not associated with an immunoglobulin heavy chain. Unlike a typical whole immunoglobulin molecule, a Free Light Chain protein is not covalently linked to an immunoglobulin heavy chain, e.g. the Free Light Chain is not disulphide bonded to a heavy chain. Typically the Free Light Chain comprises approximately 220 amino acids. Typically, the Free Light Chain protein comprises a variable region (often referred to as the Light Chain variable Region, $V_L$) and a constant region (often referred to as the Light Chain constant Region, $C_L$). Humans produce two types of immunoglobulin light chains, named with the letter kappa (κ) and lambda (λ). Each of these can be further divided into sub-groups based on variation in the variable region, with four kappa subtypes (Yκ1, Vκ2, Vκ3 and Vκ4) and six lambda subtypes (Vλ1, Vλ2, Vλ3, Vλ4, Vλ5 and Vλ6). Free Light Chain κ is typically monomeric. Free Light Chain λ is typically dimeric, linked by disulphide bonding (to another Free Light Chain λ). Polymeric forms of Free Light Chain λ and of Free Light Chain κ have been identified. Free light chains are produced by bone marrow and lymph node cells as well as locally in the periodontium by diffuse lymphocytes, and are rapidly cleared from the blood and catabolised by the kidneys. Monomeric free light chains are cleared in 2-4 hours, and dimeric free light chains in 3-6 hours.

Alpha-1-acid glycoprotein (A1AGP) is a plasma alpha-globulin glycoprotein synthesized primarily by the liver.

It is also sometimes known as Orosomucoid. It functions as a transport protein in the blood acts as a carrier of basic and neutrally charged lipohillic compounds. It is also believed to regulate the interaction between blood cells and endothelial cells.

Haemoglobin (Hb) is the iron-containing oxygen-transport metalloprotein in the red blood cells of nearly all vertebrates as well as the tissues of some invertebrates. Haemoglobin-beta (also known as beta globin, HBB, β-globin, and haemoglobin subunit beta) is a globin protein, which along with alpha globin (HBA), makes up the most common form of haemoglobin in adult humans, the HbA. Hb-β is typically 146 amino acids long and has a molecular weight of 15,867 Da. Normal adult human HbA is a heterotetramer consisting of two alpha chains and two beta chains. Hb-β is encoded by the HBB gene on human chromosome 11.

Haemoglobin subunit delta (also known as delta globin, HBD, δ-globin, and haemoglobin delta) is a globin protein, which along with alpha globin (HBA), makes up the less common form of haemoglobin in adult humans, the HbA-2. Hb-delta is typically 147 amino acids long and has a molecular weight of 16,055 Da. Adult human HbA-2 is a heterotetramer consisting of two alpha chains and two delta chains. Hb-delta is encoded by the HBD gene on human chromosome 11.

Keratin-4 (K4), also known as cytoskeletal Keratin 4 (CYK4) or cytokeratin-4 (CK-4) is a protein that in humans is encoded by the KRT4 gene. It is a member of the keratin gene family. The type II cytokeratins consist of basic or neutral proteins which are arranged in pairs of heterotypic keratin chains coexpressed during differentiation of simple and stratified epithelial tissues. The type II cytokeratin CK4 is specifically expressed in differentiated layers of the mucosal and esophageal epithelia with family member KRT13. Mutations in these genes have been associated with White Sponge Nevus, characterized by oral, esophageal, and anal leukoplakia. The type II cytokeratins are clustered in a region of chromosome 12q12-q13.

Profilin is an actin-binding protein involved in the dynamic turnover and restructuring of the actin cytoskeleton, found in most cells. It is important for spatially and temporally controlled growth of actin microfilaments, which is an essential process in cellular locomotion and cell shape changes. Human profilin-1 is typically 140 amino acids long when expressed but is often further processed into a mature form.

S100 calcium binding protein A9 (S100A9), also known as calgranulin B, is a calcium- and zinc-binding protein which plays a prominent role in the regulation of inflammatory processes and immune response. It can induce neutrophil chemotaxis, adhesion, can increase the bactericidal activity of neutrophils by promoting phagocytosis via activation of SYK, PI3K/AKT, and ERK1/2 and can induce degranulation of neutrophils by a MAPK-dependent mechanism.

When other biomarkers are optionally included, the total number of biomarkers (i.e. the biomarker panel of the invention plus other biomarkers) is typically 3, 4, 5 or 6.

However, a desirable advantage of the present invention is that the classification of periodontitis in a patient can be determined by measuring preferably not more than four biomarkers, for example two, three or four protein biomarkers. Particularly, the determination does not need to involve the use of other data, which advantageously provides a simple and straightforward diagnostic test.

The following combinations of only two protein biomarkers are thought to be particularly advantageous: MMP-8 and MMP-9; MMP-8 and PK; MMP-8 and S100A8; or MMP-9 and Pyruvate kinase.

The method, as desired, requires only that a small saliva sample, e.g. a dropsize, is taken from the subject. The size of the sample will typically range of from 0.1 μl to 2 ml, such as 1-2 ml, whereby smaller amounts, e.g., 0. 1 to 100 μl can be used for *in vitro* device processing, and whereby taking a larger sample, such as up to 20 ml, such as 7.5 to 17 ml, is also possible.

This sample is entered into an *in vitro* diagnostic device, which measures the concentration(s) of the proteins involved, and which returns a diagnostic outcome, classifying the subject on the basis of a likelihood of having periodontitis.

The ease of use of this invention will make it possible to test the majority of dental patients with periodontitis, or with a high risk for developing periodontitis, on a regular basis (e.g. as part of a regular dental check or even at home). This allows, *inter alia*, detecting the presence of periodontitis soon after it has developed, and thus enables more timely taking oral care measures to prevent its progression. Or, e.g., with patients known to be at high risk for periodontitis, and tested for the first time, the method allows to identify whether the periodontitis has developed. Particularly, the method is also suitable for self-diagnosis, whereby the steps of taking the sample and entering it into a device can be conducted by the patient him- or herself.

The patient may typically be known or suspected to have periodontitis when the invention is carried out to confirm whether the periodontitis is present. In certain embodiments therefore, the method is for assessing whether a human patient, known or suspected to have periodontitis, has periodontitis.

A method of the invention typically comprises detecting the aforementioned proteins making up a biomarker panel of the invention, and optional further biomarker proteins, by using one or more detection reagents.

The "saliva" that is tested according to the invention may be undiluted saliva, which may be obtained by spitting or swabbing, or diluted saliva, which may be obtained by rinsing the mouth with a fluid. Diluted saliva may be obtained by the patient rinsing or swilling their mouth for a few seconds with sterile water (for example 5ml or 10ml) or other suitable fluid, and spitting into a container. Diluted saliva may sometimes be referred to as an oral rinse fluid.

By "detecting" is meant measuring, quantifying, scoring, or assaying the concentration of the biomarker proteins. Methods of evaluating biological compounds, including biomarker proteins, are known in the art. It is recognized that methods of detecting a protein biomarker include direct measurements and indirect measurements. One skilled in the art will be able to select an appropriate method of assaying a particular biomarker protein.

The term "concentration" with respect to the protein biomarkers is to be given its usual meaning, namely the abundance of the protein in a volume. Protein concentration is typically measured in mass per volume, most typically mg/ml, μg/ml or ng/ml, but sometimes as low as pg/ml. An alternative measure is Molarity (or Molar concentration), mol/L or "M". The concentration can be determined by detecting the amount of protein in a sample of known, determined or pre-determined volume.

An alternative to determining the concentration is to determine the absolute amount of the protein biomarker in the sample, or determining the mass-fraction of the biomarker in the sample, for example the amount of the biomarker relative to the total of all other proteins in the sample.

A "detection reagent" is an agent or compound that specifically (or selectively) binds to, interacts with or detects the protein biomarker of interest. Such detection reagents may include, but are not limited to, an antibody, polyclonal antibody, or monoclonal antibody that preferentially binds the protein biomarker.

The phrase "specifically (or selectively) binds" or "specifically (or selectively) immunoreactive with," when referring to a detection reagent, refers to a binding reaction that is determinative of the presence of the protein biomarker in a heterogeneous population of proteins and other biologics. Thus, under designated immunoassay conditions, the specified detection reagent (e.g. antibody) binds to a particular protein at least two times the background and does not substantially bind in a significant amount to other proteins present in the sample. Specific binding under such conditions may require an antibody that is selected for its specificity for a particular protein. A variety of immunoassay formats may be used to select antibodies specifically immunoreactive with a particular protein. For example, solid-phase ELISA immunoassays (enzyme linked immunosorbent assay) are routinely used to select antibodies specifically immunoreactive with a protein (*see*, *e.g.*, Harlow & Lane, Antibodies, A Laboratory Manual (1988), for a description of immunoassay formats and conditions that can be used to determine specific immunoreactivity). Typically a specific or selective reaction will be at least twice the background signal or noise and more typically more than 10 to 100 times the background.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. This includes, e.g., Fab' and F(ab)'2 fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region. The antibody may be a bispecific antibody, e.g. an antibody that has a first variable region that specifically binds to a first antigen and a second variable region that specifically binds to a second, different, antigen. Use of at least one bispecific antibody can reduce the number of detection reagents needed.

Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive.

The biomarker protein(s) of the invention can be detected in a sample by any means. Preferred methods for biomarker detection are antibody-based assays, protein array assays, mass spectrometry (MS) based assays, and (near) infrared spectroscopy based assays. For example, immunoassays, include but are not limited to competitive and non-competitive assay systems using techniques such as Western blots, radioimmunoassays, ELISA, "sandwich" immunoassays, immunoprecipitation assays, precipitin reactions, gel diffusion precipitin reactions, immunodiffusion assays, fluorescent immunoassays and the like. Such assays are routine and well known in the art. Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1 % sodium deoxycholate, 0.1% SDS, 0.15 M NaCl, 0.01 M sodium phosphate at pH 7.2, 1 % Trasylol) supplemented with protein phosphatase and/or protease inhibitors (e.g., EDTA, PMSF, aprotinin, sodium vanadate), adding an antibody of interest to the cell lysate, incubating for a period of time (e.g., 1-4 hours) at 4°C, adding protein A and/or protein G sepharose beads to the cell lysate, incubating for about an hour or more at 4°C, washing the beads in lysis buffer and resuspending the beads in SDS/sample buffer. The ability of the antibody to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with Sepharose beads).

Western blot analysis generally comprises preparing protein samples, electrophoresis of the protein samples in a polyacrylamide gel (e.g., 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 1251) diluted in blocking buffer, washing the membrane in wash buffer, and detecting the presence of the antigen. One of skill in the art would be knowledgeable as to the parameters that can be modified to increase the signal detected and to reduce the background noise.

ELISAs typically comprise preparing antigen (i.e. the biomarker protein of interest or fragment thereof), coating the well of a 96- well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art.

Since multiple markers are used, a threshold is determined on the basis of the joint concentrations of the biomarkers (and optionally age). This threshold determines whether a patient is classified as having periodontitis or not. The invention reflects the insight that periodontitis can be detected, with sufficient accuracy based on a measurement of the combination of biomarkers as indicated above.

This insight supports another aspect, the invention, which is the use of at least two proteins selected from Matrix metalloproteinase-8 (MMP-8), Matrix metalloproteinase-9 (MMP-9), Pyruvate Kinase (PK) and S100 calcium-binding protein A8 (S100A8) as biomarkers in a saliva sample of a human patient, for assessing whether the patient has periodontitis.

This use can be implemented in a method as substantially described hereinbefore and hereinafter.

The method of the invention comprises determining a testing value reflecting the joint concentrations measured for said proteins. A joint concentration value can be any value obtained by input of the concentrations as determined and an arithmetic operation of these values. This can, e.g., be a simple addition of the concentrations. It can also involve multiplying each concentration with a factor reflecting a desired weight of these concentrations, and then adding up the results. It can also involve multiplying the concentrations with each other, or any combination of multiplication, division, subtraction, exponentiation, and addition. It can further involve raising concentrations to some power.

Optionally, the testing value reflects the concentration of joint concentrations determined for said protein(s) in combination with the age of the subject.

The resulting joint concentration value is compared with a threshold value reflecting in the same manner the joint concentrations associated with the presence of periodontitis. The comparison allows assessing whether the testing value is indicative of the presence of periodontitis in the patients whose saliva is subjected to the test.

The threshold value can, e.g., be based on the joint concentration value, obtained in the same manner on the basis of the concentration(s) determined for the same protein(s) in a reference sample associated with the presence of periodontitis, i.e. in a patient diagnosed with periodontitis. Typically, thereby a value reflecting the same or higher joint concentration is indicative of the presence of periodontitis in a tested patient. Analogously, a value reflecting a lower joint concentration in the saliva of a tested periodontitis patient, indicates that periodontitis is absent. However, it will be understood that it is also possible to calculate a threshold value (e.g. by using a negative multiplier) such that a testing value indicating periodontitis would be below the threshold, and a testing value indicating absence of periodontitis, would be above the threshold.

The threshold value can also be determined on the basis of measuring the concentration(s) of the present biomarker protein(s) in a set of samples, including patients with a known diagnosis of periodontitis and "not" periodontitis. Thereby the measured concentration values can be subjected to statistical analysis, possibly including machine learning methods, allowing to discriminate, with the desired sensitivity and specificity, patients classified as periodontitis and patients classified as not suffering from periodontitis. Therefrom, the desired threshold value can be obtained. On the basis of this threshold value, a sample to be tested can be subjected to the same concentration measurement, and the concentration values are then processed, in the same manner in which the threshold value is obtained, so as to determine a joint concentration value that can be compared with the threshold, thus allowing the tested sample to be classified as having periodontitis or not.

In an interesting embodiment, the joint concentration value is obtained in the form of a score as follows. A numerical value (protein concentration values in e.g. ng/ml) is assigned to each measurement, and these values are used in a linear or non-linear combination to calculate a score between zero and one. In the event that the threshold value is determined on the basis of a set of subjects as mentioned above, the score between 0 and 1 is typically calculated with the sigmoid function that takes the joint concentration as input (as shown further on).

When the score exceeds a certain threshold, the method indicates that the patient has periodontitis. The threshold may be chosen based on the desired sensitivity and specificity.

It will be understood that in performing a 'periodontitis classification' on a subject, in accordance with the invention, this can be on subjects for which there is no knowledge or awareness of their periodontitis status, or on subjects that can be assumed to be at risk from, or suffering from, periodontitis. This prior knowledge can typically either be known from e.g. a previously performed diagnosis of periodontitis, though perhaps without ability to differentiate the extent of it, or, e.g., assumed from the subject's oral health condition record.

Clinical definitions as acknowledged in the art are based on the following:

*Gingival Index (GI)*

A full mouth gingival index will be recorded based on the Lobene Modified Gingival Index (MGI) rated on a scale of 0 to 4, where:

- 0 = absence of inflammation,
- 1 = mild inflammation; slight change in color little change in texture of any portion of but not the entire margin or papillary gingival unit,
- 2 = mild inflammation; but involving entire margin or papillary unit,
- 3 = moderate inflammation; glazing, redness, oedema and/or hypertrophy of margin or papillary unit,
- 4 = severe inflammation; marked redness, oedema and/or hypertrophy of marginal or papillary gingival unit, spontaneous bleeding, congestion, or ulceration].

*Probing depths (PD)*

Probing depths will be recorded to the nearest mm using a manual UNC-15 periodontal probe. Probing depth is the distance from the probe tip (assumed to be at the base of the pocket) to the free gingival margin.

*Gingival recession (REC)*

Gingival recession will be recorded to the nearest mm using a manual UNC-15 periodontal probe. Gingival recession is the distance from the free gingival margin to the cemento-enamel junction. Gingival recession will be indicated as a positive number and gingival overgrowth will be indicated as a negative number.

*Clinical attachment loss (CAL)*

Clinical attachment loss will be calculated as the sum of probing depth + recession at each site.

*Bleeding on probing (BOP)*

Following probing, each site will be assessed for bleeding on probing, if bleeding occurs within 30s of probing, a score of 1 will be assigned for the site, otherwise a score of 0 will be assigned.

The resulting subject group (patient group) definition is as follows, whereby the mild-moderate periodontitis and the advanced periodontitis groups are "periodontitis" relevant to the present invention:

- Healthy group (H): PD $\leq$ 3 mm in all sites (but would allow up to four 4 mm pockets at distal of last standing molars), no sites with interproximal attachment loss, GI of $\geq$ 2.0 in $\leq$ 10% sites, %BOP scores $\leq$ 10%;
- Gingivitis group (G): GI $\geq$ 3.0 in > 30% of sites, no sites with interproximal attachment loss, no sites with PD > 4 mm , %BOP scores > 10%;
- Mild-moderate periodontitis group (MP): interproximal PD of 5-7 mm, (equating to approximately 2-4 mm CAL) at $\geq$ 8 teeth, %BOP scores > 30%;
- Advanced periodontitis group (AP): interproximal PD of $\geq$ 7 mm, (equating to approximately $\geq$ 5 mm CAL) at $\geq$ 12 teeth, %BOP scores > 30%.

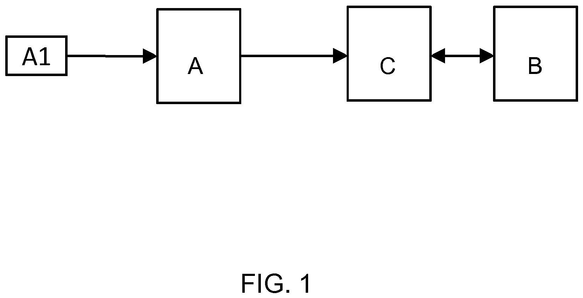

In an embodiment, the method of the invention makes use of a system as represented schematically in Fig. 1. The system can be a single apparatus having various device components (units) integrated therein. The system can also have its various components, or some of these components, as separate apparatuses. The components shown in Fig. 1 are a measurement device (A), a graphical user interface (B) and a computer processing unit (C).

As mentioned above, the system of the invention comprises a data connection to an interface, whereby the interface itself can be a part of the system or can be a remote interface. The latter refers to the possibility to use a different apparatus, preferably a handheld apparatus such as a smartphone or a tablet computer, for providing the actual interface. The data connection in such cases will preferably involve wireless data transfer such as by Wi-Fi or Bluetooth, or by other techniques or standards.

The measurement device (A) is configured to receive a saliva sample, for example by putting a drop of saliva on a cartridge (A1), which can be inserted into the device (A). The device can be an existing device that is capable to determine, from the same saliva sample, the concentrations of the proteins.

The processing unit (C) receives numerical values for the protein concentrations from part (A). The unit (C) is provided with software (typically embedded software) allowing it to calculate a score (S) between 0 and 1. The software further includes a numerical value for the threshold (T). If the calculated value (S) exceeds (T), unit (C) will output an indication (I) of 'periodontitis' to the GUI (B), otherwise it will output 'not periodontitis'. A further embodiment may use the specific value of (S) to indicate the certainty with which the indication (I) is made. This can be a probability score, whereby 0.5 is a possible threshold value, and e.g. a score S=0.8 would indicate the probability of periodontitis. Interesting options are:

Based on the score S, one can directly indicate a certainty, i.e. S=0.8 means 80% certainty of periodontitis; or
To make the indication through the definition of a range R1-R2, such that when R1<S<R2, the indication (I) will read 'inconclusive'.

A specific calculation of the score can be implemented, e.g., by means of a sigmoid function applying the following formula:

$$S = \frac{1}{1 + \exp(-(c_0 + \sum_{i=1}^{N} c_i B_i))}$$

Wherein $N$ is the number of proteins/biomarkers used. $c_0$, $c_1$, etc. are coefficients (numerical values) and $B_1, B_2$, etc. are the respective protein concentrations.

Determining of the coefficients $c_i$ can be done by a training procedure:

- Select N1 subjects with periodontitis (as identified by a dentist via the current criteria) and N2 subjects without periodontitis (having healthy gums or gingivitis).
- Take a saliva sample from each subject and determine the protein concentrations of a combination of biomarkers as explained above.
- Define the score S to be 1 for periodontitis, and 0 for non-periodontitis (healthy gums or gingivitis).
- Fit the sigmoid function to the scores and protein concentration values.

Other regression or machine learning methods (linear regression, neural network, support vector machine) may be used where the score S, is high for periodontitis patients and low for the non-periodontitis/healthy controls.

In particular, such a procedure has been applied (in the Example) using a clinical study with subjects having either periodontitis or not having periodontitis (i.e. having healthy gums or gingivitis, identified by clinical assessment by a dental professional via current criteria, e.g. American Academy of Periodontology criteria). Performance of various biomarker combinations were evaluated by means of Leave-1-out cross validation, resulting in the preferred biomarker combinations of the invention.

With reference to the aforementioned system, the invention also provides, in a further aspect, a system for assessing whether a human patient has periodontitis, the system comprising:

- detection means able and adapted to detect in a sample of saliva of the human patient at least two proteins selected from Matrix metalloproteinase-8 (MMP-8), Matrix metalloproteinase-9 (MMP-9), Pyruvate Kinase (PK) and S100 calcium-binding protein A8 (S100A8);

as explained above, such means are known, and easily accessible to the skilled person. Typically, there is provided a container for receiving an oral sample of a subject therein, the container provided with the detection means;

- a processor able and adapted to determine from the determined concentrations of said proteins an indication of the patient having periodontitis.

Optionally, the system comprises a user interface (or a data connection to remote interface), particularly a graphical user interface (GUI), capable of presenting information; a GUI is a type of user interface that allows users to interact with electronic devices through graphical icons and visual indicators such as secondary notation, instead of text-based user interfaces, typed command labels or text navigation (none of such interface types being excluded in the present invention); GUIs are generally known, and are used typically in handheld mobile devices such as MP3 players, portable media players, gaming devices, smartphones and smaller household, office and industrial controls; as said, the interface optionally can also be chosen so as to be capable of putting in information, such as, e.g., the age of the subject, sex, BMI (Body Mass Index).

The invention also provides, either separately or as part of the aforementioned system, a kit for detecting at least two biomarkers for periodontitis in a sample of saliva of a human patient, said kit comprising one or more detection reagents for detecting at least two proteins selected from Matrix metalloproteinase-8 (MMP-8), Matrix metalloproteinase-9 (MMP-9), Pyruvate Kinase (PK) and S100 calcium-binding protein A8 (S100A8).

Typically, the kit comprises two or more, or three or more, detection reagents, each directed to a different biomarker of the invention. In one embodiment, a first detection reagent is for detecting MMP8 and a second detection reagent is for detecting MMP-9, PK or S100A8. A third detection reagent may be included for detecting a different one of MMP-9, PK or S100A8. A fourth detection reagent may be included for detecting the final one of MMP-9, PK and S100A8.

As discussed above with reference to the method of the invention, the kit may comprise more detection reagents, such as for other proteins. In a preferred embodiment the detection reagents made available in the kit consist of the detection reagents for the detection of two, three or four proteins making up a biomarker panel of the invention, as mentioned. In further embodiments, separate detection reagents may also be included for one of more of IL-1β, HGF, FLC Kappa, FLC Lambda, A1AGP, Hb-beta, Hb-delta, Keratin 4, Profilin, and S100A9. In further embodiments, separate detection reagents are provided for each of the biomarker proteins present in a combination exemplified in Table 1 in the Example below.

Preferably said kits comprise a solid support, such as a chip, a microtiter plate or a bead or resin comprising said detection reagents. In some embodiments, the kits comprise mass spectrometry probes, such as ProteinChip™.

The kits may also provide washing solutions and/or detection reagents specific for either unbound detection reagent or for said biomarkers (sandwich type assay).

In an interesting aspect, the recognition of a biomarker panel of the invention is applied in monitoring the status of periodontitis in a human patient, over time. Accordingly, the invention also provides an *in vitro* method for determining a change in status of periodontitis in a human patient suffering from periodontitis over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of at least two proteins selected from Matrix metalloproteinase-8 (MMP-8), Matrix metalloproteinase-9 (MMP-9), Pyruvate Kinase (PK) and S100 calcium-binding protein A8 (S100A8), and comparing the concentrations, whereby a difference of preferably at least two concentrations, reflects a change in status. This difference can be reviewed as a difference in concentrations, thus allowing a direct comparison without first generating a number between 0 and 1, or any other classification. It will be understood that the measurements received at both points in time can also be processed in just the same manner as done when determining the periodontitis status as above.

The invention also provides a method of diagnosing whether a human patient has periodontitis, comprising detecting in a sample of saliva of the human patient at least two proteins selected from Matrix metalloproteinase-8 (MMP-8), Matrix metalloproteinase-9 (MMP-9), Pyruvate Kinase (PK) and S100 calcium-binding protein A8 (S100A8).

The presence of periodontitis in the patient is typically assessed on the basis of the concentrations of said proteins in said sample. Optionally, the method of this aspect comprises the further step of treating the periodontitis in the patient. This optional treatment step can comprise the administration of known therapeutic agents or dental procedures, or a combination of therapeutic agents and dental procedures. Known therapeutic agents include the administration of antimicrobial-containing agents such as a mouthwash, chip, gel or microsphere. A typical antimicrobial agent for use in treating periodontitis is chlorhexidine. Other therapeutic agents include antibiotics, typically orally-administered antibiotics, and enzyme suppressants such as doxycycline. Known non-surgical therapeutic procedures include scaling and root planing (SRP). Known surgical procedures include surgical pocket reduction, flap surgery, gum grafts or bone grafts, which are typically reserved for advanced periodontitis and not typically used to treat gingivitis.

The invention further provides a method of detecting at least two proteins selected from Matrix metalloproteinase-8 (MMP-8), Matrix metalloproteinase-9 (MMP-9), Pyruvate Kinase (PK) and S100 calcium-binding protein A8 (S100A8);

in a human patient, comprising:

(a) obtaining a saliva sample from a human patient; and
(b) detecting whether the proteins are present in the sample by contacting the sample with one or more detection reagents for binding said proteins and detecting binding between each protein and the one or more detection reagents.

The invention will be further illustrated with reference to the following nonlimiting example.

Example

A clinical study was carried out with 153 subjects, of whom:

39 had healthy gums (H)
35 were diagnosed with gingivitis (G)
41 were diagnosed with mild periodontitis (MP)
38 were diagnosed with advanced periodontitis (AP).

Receiver-Operator-Characteristic Area-Under-the Curve values of >0.9 were obtained using the biomarker panels of the invention containing a maximum of 4 protein biomarkers.

ROC (Receiver-Operator-Characteristic) Area-Under-the Curve (AUC) values were obtained. Performance of various biomarker combinations were evaluated by means of logistic regression with leave-one-out cross validation (LOOCV), resulting in the preferred biomarker combinations as explained herein.

In statistics, a receiver operating characteristic curve, or ROC curve, is a graphical plot that illustrates the performance of a binary classifier system as its discrimination threshold is varied. The curve is created by plotting the true positive rate (TPR) against the false positive rate (FPR) at various threshold settings. The true-positive rate is also known as sensitivity, recall or *probability of detection* in machine learning. The false-positive rate is also known as the fall-out or *probability of false alarm* and can be calculated as (1 - specificity). The ROC curve is thus the sensitivity as a function of fall-out. In general, if the probability distributions for both detection and false alarm are known, the ROC curve can be generated by plotting for every value of the threshold, the value of the cumulative distribution function (area under the probability distribution from - $\infty$ to the discrimination threshold) of the detection probability on the y-axis, versus the value of the cumulative distribution function of the false-alarm probability on the x-axis. The accuracy of the test depends on how well the test separates the group being tested into those with and without the disease in question. Accuracy is measured by the area under the ROC curve. An area of 1 represents a perfect test; an area of 0.5 represents a worthless test. A guide for classifying the accuracy of a diagnostic test is the traditional academic point system:

- 0.90-1 = excellent (A)
- 0.80-0.90 = good (B)
- 0.70-0.80 = fair (C)
- 0.60-0.70 = poor (D)
- 0.50-0.60 = fail (F)

Based on the foregoing, in the results of the aforementioned clinical study, an ROC AUC value of above 0.75 is considered to represent a desirable accuracy for providing a diagnostic test.

The protein biomarkers explored in this study are:

- MMP8

- MMP9

- IL-1$\beta$

- HGF

- Free Light Chain (FLC) $\kappa$ (kappa)

- Free light chain (FLC) $\lambda$ (lambda)

- A1AGP

- Hb-beta

- Hb-delta

- Keratin 4

- Profilin

- Pyruvate Kinase

- S100A8

- S100A9

Furthermore, in the employed logistic regression we considered as additional predictors $\kappa+\lambda$, $\kappa-\lambda$, $\kappa/\lambda$.

Additionally we included age as predictor.

This yields a total number of 4204 possible non-redundant panels, having at most 4 protein biomarkers (panel having only age is not considered). Non-redundant here means that a panel including e.g. $\kappa+\lambda$ and $\kappa-\lambda$ as predictors is not considered, as in the logistic regression it gives the same result as the corresponding panel including $\kappa$ and $\lambda$ as predictors.

Note that not restricting the number of protein markers in a panel, yields a number of 98302 possible non-redundant panels (panel having only age is not considered) given the predictors mentioned above.

From this study, 1972 panels having at most 4 protein markers were found to give AUC LOOCV>0.9.

Of these panels:

- 4 have one protein marker (MMP8, or MMP9, both plus optionally age)
- 59 have two protein markers
- 368 have three protein markers
- 1541 have four protein markers

It can be seen that only MMP-8 and MMP-9 (plus possible age) give AUC>0.9 on their own, otherwise at least two marker panels are needed for AUC>0.9.

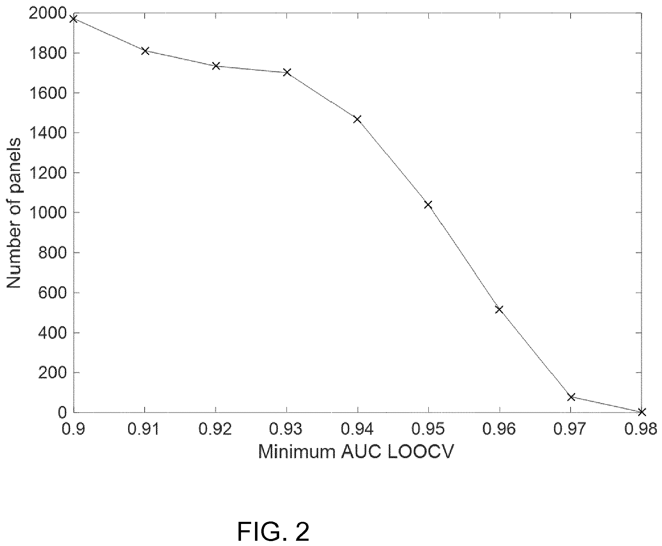

The number of panels versus required performance, in terms of minimum AUC LOOCV, is shown in Fig. 2.

The top performing ~150 panels having at most four protein biomarkers with AUC>~0.97, have at least two markers from MMP8, MMP9, Pyruvate kinase, and S100A8. Biomarker combinations comprising two, three or four of MMP8, MMP9, Pyruvate kinase, and S100A8 are therefore preferred according to the invention.

Note that using the single marker panels MMP8, or MMP9 (plus optionally age) give an AUC LOOCV between 0.94 and 0.97. In that respect only the top performing panels with AUC>0.97 may be preferred.

Of these best performing panels (AUC>0.97), four have 2 markers, 29 have 3 markers, and the rest have 4 markers. The 4 panels having only 2 markers are:

MMP8+MMP9
MMP8+Pyruvate kinase
MMP8+S100A8
MMP9+Pyruvate kinase.

Note that these top performing panels in fact have at least one of MMP8 and MMP9. These four two-marker panels are preferred embodiments of the invention.

The top 25 performing panels with at most 4 protein biomarkers are provided in Table 1 below, having an AUC>0.97:

| MMP8 | IL1B | MMP9 | HGF | Age | κ | λ | κ+λ | κ/λ | κ-λ | A1AGP | Hb-beta | Hb-delta | Keratin 4 | Profilin | Pyruvate Kinase | S100A8 | S100A9 | AUC LOOCV |
|---|---|---|---|---|---|---|---|---|---|---|---|---|---|---|---|---|---|---|
| X |  | X |  | X |  |  |  |  |  |  |  |  |  |  | X |  |  | 0.975 |
| X |  | X |  | X | X |  |  |  |  |  |  |  |  |  | X |  |  | 0.976 |
| X |  | X |  | X |  | X |  |  |  |  |  |  |  |  | X |  |  | 0.975 |
| X |  |  |  | X | X |  |  | X |  |  |  |  |  |  | X |  |  | 0.974 |
| X |  |  |  | X |  |  | X | X |  |  |  |  |  |  | X |  |  | 0.975 |
| X |  | X |  | X |  |  |  |  |  |  |  | X |  |  | X |  |  | 0.975 |
| X |  | X |  | X |  |  |  |  |  |  |  |  | X |  | X |  |  | 0.974 |
| X |  | X |  | X |  |  |  |  |  |  |  |  |  | X | X |  |  | 0.974 |
| X |  | X |  | X |  |  |  |  |  |  |  |  |  |  |  | X |  | 0.975 |
| X | X | X |  | X |  |  |  |  |  |  |  |  |  |  |  | X |  | 0.974 |
| X |  | X |  | X | X |  |  |  |  |  |  |  |  |  |  | X |  | 0.974 |
| X |  | X |  | X |  |  |  |  |  | X |  |  |  |  |  | X |  | 0.974 |
| X |  | X |  | X |  |  |  |  |  |  |  | X |  |  |  | X |  | 0.976 |
| X |  |  |  | X |  |  |  |  |  |  |  |  |  |  |  | X | X | 0.979 |
| X | X |  |  | X |  |  |  |  |  |  |  |  |  |  |  | X | X | 0.977 |
| X |  | X |  | X |  |  |  |  |  |  |  |  |  |  |  | X | X | 0.979 |
| X |  |  | X | X |  |  |  |  |  |  |  |  |  |  |  | X | X | 0.976 |
| X |  |  |  | X | X |  |  |  |  |  |  |  |  |  |  | X | X | 0.981 |
| X |  |  |  | X |  | X |  |  |  |  |  |  |  |  |  | X | X | 0.977 |
| X |  |  |  | X |  |  |  |  |  | X |  |  |  |  |  | X | X | 0.977 |
| X |  |  |  | X |  |  |  |  |  |  | X |  |  |  |  | X | X | 0.979 |
| X |  |  |  | X |  |  |  |  |  |  |  | X |  |  |  | X | X | 0.981 |
| X |  |  |  | X |  |  |  |  |  |  |  |  | X |  |  | X | X | 0.976 |
| X |  |  |  | X |  |  |  |  |  |  |  |  |  | X |  | X | X | 0.976 |
| X |  |  |  | X |  |  |  |  |  |  |  |  |  |  |  | X | X | 0.979 |

**Table 1**

Each of the protein biomarker combinations in this table is highlighted as a preferred combination of the invention. Age may or may not be included as an additional biomarker.

While the invention has been illustrated and described in detail in the drawings and foregoing description, such illustration and description are to be considered illustrative or exemplary and not restrictive; the invention is not limited to the disclosed embodiments. For example, it is possible to present detection reagents for different biomarkers in different units. Or, conveniently, a kit of the invention can comprise a fixed set of detection reagents for the protein biomarkers that are used in most embodiments, e.g. MMP-8, MMP-9, PK or S100-A8 and optionally flexible modules comprising a detection reagent for other biomarkers such as IL-1β, HGF, FLC Kappa, FLC Lambda, A1AGP, Hb-beta, Hb-delta, Keratin 4, Profilin, and/or S100A9.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum, we hereby disclose an *in vitro* method for assessing whether a human patient is suffering from periodontitis. The method is based on the insight to determine biomarker proteins. Accordingly, in a sample of saliva from a patient, the concentrations are measured of the proteins described herein. Based on the concentrations as measured, a value is determined reflecting the joint concentrations for said proteins. This value is compared with a threshold value reflecting in the same manner the joint concentrations associated with periodontitis. The comparison allows assessing whether the testing value is indicative of the presence of periodontitis in said patient. Thereby, typically, a testing value reflecting a joint concentration below the joint concentration reflected by the threshold value is indicative for the absence of periodontitis in said patient, and a testing value reflecting a joint concentration at or above the joint concentration reflected by the threshold value, is indicative for periodontitis in said patient.

**Claims**

1.   An *in vitro* method for assessing whether a human patient has periodontitis, wherein the method comprises:

   - detecting, in a sample of saliva from said human patient, the concentrations of at least two proteins selected from Matrix metalloproteinase-8 (MMP-8), Matrix metalloproteinase-9 (MMP-9), Pyruvate Kinase (PK) and S100 calcium-binding protein A8 (S100A8);
   - determining a testing value reflecting the joint concentrations determined for said proteins;
   - comparing said testing value with a threshold value reflecting in the same manner the joint concentrations associated with periodontitis, so as to assess whether the testing value is indicative for periodontitis in said patient.

2.   A method according to claim 1, wherein the human patient is suspected to have periodontitis.

3.   A method according to claim 1 or claim 2, wherein the age of the subject is determined and the testing value reflects the joint concentrations determined for said proteins, in combination with the age of the subject.

4.   A method according to any preceding claim, wherein the threshold value is based on the concentrations determined for the proteins in one or more reference samples each sample associated with the presence of periodontitis or absence of periodontitis.

5.   A method according to any of claims 1 to 3, wherein the threshold value is based on the concentrations of the proteins in a set of samples, including samples from subjects that have periodontitis and samples from subjects not having periodontitis.

6.   A method according to any one of the preceding claims, wherein the proteins comprise MMP-8 and/or MMP-9.

7.   A method according to any one of the preceding claims, wherein the proteins comprise: MMP-8 and MMP-9; MMP-8 and PK; MMP-8 and S100A8; or MMP-9 and Pyruvate kinase.

8.   A method according to any one of the preceding claims, wherein the proteins additionally comprise one or more of IL-1β, HGF, FLC Kappa, FLC Lambda, A1AGP, Hb-beta, Hb-delta, Keratin 4, Profilin, and S100A9.

9.   A method according to any one of the claims 1 to 6, wherein the proteins comprise or consist of Matrix metalloproteinase-8 (MMP-8), Matrix metalloproteinase-9 (MMP-9), Pyruvate Kinase (PK) and S100 calcium-binding protein A8 (S100A8).

10. A method according to any one of the preceding claims, wherein the concentration values determined are arithmetically processed into a number between 0 and 1.

11. The use of two or more of the proteins Matrix metalloproteinase-8 (MMP-8), Matrix metalloproteinase-9 (MMP-9), Pyruvate Kinase (PK) and S100 calcium-binding protein A8 (S100A8) in a sample of saliva of a human patient, as biomarkers for assessing whether the patient has periodontitis.

12. The use according to claim 11, wherein the age of the human patient is also used as a biomarker.

13. A system for assessing whether a human patient has periodontitis, the system comprising:

- detection means able and adapted to detect in a sample of saliva of the human patient two or more of the proteins Matrix metalloproteinase-8 (MMP-8), Matrix metalloproteinase-9 (MMP-9), Pyruvate Kinase (PK) and S100 calcium-binding protein A8 (S100A8);
- a processor able and adapted to determine from the determined concentrations of said proteins an indication of the patient having periodontitis.

14. A system according to claim 13, further comprising a container for receiving an oral fluid sample, the container comprising the detection means.

15. A system according to claim 13 or 14, further comprising:

- a user interface for presenting the indication to a user; and
- a data connection between the processor and the user interface for transferring the indication from the processor to the user interface.

16. A system according to any one of claims 13 to 15, wherein the processor is enabled to function by means of an internet-based application.

17. A system according to any of claims 13 to 16, wherein the interface is capable of putting in information on the age of the subject and the processor is able and adapted to determine from the determined concentrations, an indication that the patient has periodontitis.

18. A kit for detecting at least two biomarkers for periodontitis in a sample of saliva of a human patient, said kit comprising one or more detection reagents for detecting two or more of the proteins Matrix metalloproteinase-8 (MMP-8), Matrix metalloproteinase-9 (MMP-9), Pyruvate Kinase (PK) and S100 calcium-binding protein A8 (S100A8).

19. A kit according to claim 18, wherein the one or more detection reagents comprise a first detection reagent for detecting MMP-8, a second detection reagent for detecting MMP-9, a third detection reagent for detecting PK and a fourth detection reagent for detecting S100A8.

20. A kit according to claim 18 or 19, wherein the one or more detection reagents are contained on a solid support.

21. A kit according to any one of the claims 18 to 20, wherein the one or more detection reagents consist of a first detection reagent for detecting MMP-8, a second detection reagent for detecting MMP-9, a third detection reagent for detecting PK and a fourth detection reagent for detecting S100A8.

22. An *in vitro* method for determining a change in status of periodontitis in a human patient suffering from periodontitis over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of two or more of the proteins Matrix metalloproteinase-8 (MMP-8), Matrix metalloproteinase-9 (MMP-9), Pyruvate Kinase (PK) and S100 calcium-binding protein A8 (S100A8), and comparing the concentrations, whereby a difference in at least one of the concentrations, reflects a change in status.

23. A method of diagnosing whether a human patient has periodontitis, comprising detecting in a sample of saliva of the human patient two or more of the proteins Matrix metalloproteinase-8 (MMP-8), Matrix metalloproteinase-9 (MMP-9), Pyruvate Kinase (PK) and S100 calcium-binding protein A8 (S100A8) and assessing the presence of periodontitis in the patient on the basis of the concentrations of said proteins in said sample.

24. A method of detecting two or more of the proteins Matrix metalloproteinase-8 (MMP-8), Matrix metalloproteinase-9 (MMP-9), Pyruvate Kinase (PK) and S100 calcium-binding protein A8 (S100A8), comprising:

(a) obtaining a saliva sample from a human patient; and
(b) detecting whether the two or more proteins are present in the sample by contacting the sample with one or more detection reagents for binding said proteins and detecting binding between each protein and the one or more detection reagents.

FIG. 1

FIG. 2

Europäisches
Patentamt

European
Patent Office

Office européen
des brevets

# EUROPEAN SEARCH REPORT

Application Number

EP 18 16 6975

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/018342 A1 (UNIV MICHIGAN [US]; BRAUN THOMAS [US]; GIANNOBILE WILLIAM V [US]; HERR) 5 February 2009 (2009-02-05) * par 0089, 0090, 0095, cl 1-5, 10-12, 18-20, 22. * | 1-13,17, 18,20-24 | INV. G01N33/573 G01N33/68 G06F19/10 G16H50/30 |
| X | CHRISTOPH A. RAMSEIER ET AL: "Identification of Pathogen and Host-Response Markers Correlated With Periodontal Disease", JOURNAL OF PERIODONTOLOGY., vol. 80, no. 3, 1 March 2009 (2009-03-01), pages 436-446, XP055400653, US ISSN: 0022-3492, DOI: 10.1902/jop.2009.080480 * pg 438, right col, third par-pg 439, first par; 439, last par-pg 441; tables 3 and 4. * | 1-13, 18-20, 22-24 | |
| X | Timo Sorsa ET AL: "effetcs of tetracyclines on Neutrophil, Gingival, and Salivary Collagenases", , 1 January 1998 (1998-01-01), XP055476623, Retrieved from the Internet: URL:https://nyaspubs.onlinelibrary.wiley.com/doi/epdf/10.1111/j.1749-6632.1994.tb24729.x [retrieved on 2018-05-17] * pg 113, second par, pg 114, last par-pg 115, first pat; fig 7. * | 1,2,6, 11,23,24 | TECHNICAL FIELDS SEARCHED (IPC) G01N G06F G16H |
| X | US 2017/199189 A1 (WADE JILL [US]) 13 July 2017 (2017-07-13) * par 0140-0141, 0155, 0158; cl 5. * | 13-17 | |

-/--

~~The present search report has been drawn up for all claims~~

| Place of search | Date of completion of the search | Examiner |
|---|---|---|
| Munich | 24 May 2018 | Motrescu-Hateley, E |

EPO FORM 1503 03.82 (P04C01)

Europäisches
Patentamt

European
Patent Office

Office européen
des brevets

## EUROPEAN SEARCH REPORT

Application Number

EP 18 16 6975

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/046465 A1 (LAMBERT REBECCA [US]) 12 February 2015 (2015-02-12) * par 0038, 0041, 0055; cl 1-13. * | 13-17 | |

TECHNICAL FIELDS
SEARCHED        (IPC)

~~The present search report has been drawn up for all claims~~

| Place of search | Date of completion of the search | Examiner |
|---|---|---|
| Munich | 24 May 2018 | Motrescu-Hateley, E |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

Europäisches
Patentamt

European
Patent Office

Office européen
des brevets

**Application Number**

EP 18 16 6975

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-24(partially)

☐ The present supplementary European search report has been drawn up for those parts of the European patent application which relate to the invention first mentioned in the claims (Rule 164 (1) EPC).

| | Europäisches Patentamt European Patent Office Office européen des brevets | **LACK OF UNITY OF INVENTION SHEET B** | Application Number EP 18 16 6975 |
|---|---|---|---|

The Search Division considers that the present European patent application does not comply with the requirements of unity of invention and relates to several inventions or groups of inventions, namely:

1. claims: 1-24(partially)

A method for assessing whether a human patient has periodontitis, wherein the method comprises: detecting, in a sample of saliva from said human patient, the concentrations of at least two proteins selected from Matrix metalloproteinase-8 (MMP-8), Matrix metalloproteinase-9 (MMP-9), Pyruvate Kinase (PK) and S100 calcium-binding protein A8 (S100A8); determining a testing value reflecting the joint concentrations determined for said proteins; comparing said testing value with a threshold value reflecting in the same manner the joint concentrations associated with periodontitis, so as to assess whether the testing value is indicative for periodontitis in said patient. The two proteins analysed are MMP-8 and MMP-9.
---

2. claims: 1-24(partially)

A method for assessing whether a human patient has periodontitis, wherein the method comprises: detecting, in a sample of saliva from said human patient, the concentrations of at least two proteins selected from Matrix metalloproteinase-8 (MMP-8), Matrix metalloproteinase-9 (MMP-9), Pyruvate Kinase (PK) and S100 calcium-binding protein A8 (S100A8); determining a testing value reflecting the joint concentrations determined for said proteins; comparing said testing value with a threshold value reflecting in the same manner the joint concentrations associated with periodontitis, so as to assess whether the testing value is indicative for periodontitis in said patient. The two proteins analysed are the rest combinations of two proteins as disclosed in claim 1.
---

## ANNEX TO THE EUROPEAN SEARCH REPORT
## ON EUROPEAN PATENT APPLICATION NO.

EP 18 16 6975

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2018

| Patent document cited in search report | | Publication date | Patent family member(s) | | Publication date |
|---|---|---|---|---|---|
| WO 2009018342 | A1 | 05-02-2009 | US | 2010196941 A1 | 05-08-2010 |
| | | | WO | 2009018342 A1 | 05-02-2009 |
| US 2017199189 | A1 | 13-07-2017 | CA | 2952630 A1 | 30-12-2015 |
| | | | EP | 3161481 A1 | 03-05-2017 |
| | | | US | 2017199189 A1 | 13-07-2017 |
| | | | WO | 2015200898 A1 | 30-12-2015 |
| US 2015046465 | A1 | 12-02-2015 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RAMSEIER et al.** *J Periodontol.,* March 2009, vol. 80 (3), 436-46

- **HARLOW ; LANE.** Antibodies, A Laboratory Manual. 1988